# EUROPEAN PATENT SPECIFICATION

(11) **EP 3 441 075 B1**
(45) Date of publication and mention of the grant of the patent: **01.09.2021**
(21) Application number: 17778657.1
(22) Date of filing: 05.04.2017
(51) Int. Cl.: A61K 36/752, A61K 36/287, A61K 36/14, A61K 36/53, A61K 36/00, A61P 1/16, A61P 7/02, A61P 9/00, A61P 9/10, A61K 47/12, A61K 31/015, A61K 31/045, A61K 31/05, A23L 33/10

(54) **ORALLY ADMINISTERED ESSENTIAL OIL COMPOSITION AND USE THEREOF**
ORAL VERABREICHTE ZUSAMMENSETZUNG AUS ÄTHERISCHEN ÖLEN UND VERWENDUNG DAVON
COMPOSITION D'HUILE ESSENTIELLE ADMINISTRÉE PAR VOIE ORALE ET SON UTILISATION

(30) Priority: 05.04.2016 HK 16103839
(43) Date of publication of application: 13.02.2019
(73) Proprietor: Lai, Lily, Wanchai, Hong Kong (CN)
(72) Inventor: Lai, Lily, Wanchai, Hong Kong (CN)
(74) Representative: Ward, David Ian
(86) International application number: PCT/CN2017/079501
(87) International publication number: WO 2017/173993

(56) References cited:
- EP-A1- 2 878 303
- WO-A1-2015/014497
- CN-A- 1 475 204
- CN-A- 101 420 848
- CN-A- 102 058 892
- CN-A- 102 481 324
- CN-A- 103 316 108
- CN-A- 103 463 549
- DE-A1-102011 078 432
- WANG, SHANYI: "Sedative-hypnotic Efficacy of the Blended Essential Oil and Its Application Research", Dissertation, 15 October 2010 (2010-10-15), pages 1-75, XP009515754,

## Description

### TECHNICAL FIELD

The present invention relates to an oral pharmaceutical composition comprising an essential oil. The invention also relates to treating and/or preventing a disease in a subject. The invention further relates to a food supplement composition comprising an essential oil.

### BACKGROUND

Scientific reports and clinical observations have revealed that excess intake of food containing pro-inflammatory substances may result in chronic inflammation-related diseases. The pro-inflammatory substances include arachidonic acid which promotes inflammation in our bodies by generating pro-inflammatory leukotrienes. These substances predispose our bodies towards inflammation. The patients who have inappropriate food consumptions with considerable amount of pro-inflammatory substances were found to suffer from chronic diseases such as liver diseases and cardiovascular diseases. The related diseases usually include atherosclerosis, arterial embolism, arthritis, liver malfunction, asthma etc. However, the current pharmaceutical approaches such as non-steroidal anti-inflammatory drugs possess a huge risk of undesirable side-effects.

Essential oils are commonly used in aromatherapy for topical application or inhalation for stress relief and boosting immunity. Compositions comprising essential oils are known from EP2878303, EP2322145, CN103463549 amongst many others. Although there are some safety concerns on using essential oils such as when the application of an undiluted essential oil may cause irritations to the skin of a user, it is undeniable that essential oils possess significant antioxidant properties.

Accordingly, there remains a strong need for developing a pharmaceutical composition and a use in treating and/or preventing diseases, especially for those diseases accompanied by an inflammatory responses. It has been the experience of the present inventor that an essential oil may reduce skin inflammation and therefore the inventor realized that an essential oil is a potential candidate for combating the chronic inflammation-related diseases, especially for those diet-induced inflammations. There also exists a strong need for a food supplement composition which improves the health condition of a subject who lacks certain nutrients from his/her daily food source and/ or a subject who has a frequent diet with pro-inflammatory substances. There is also a need for a food supplement composition that may serve as a means to prevent a subject from suffering an inflammation-related disease.

### SUMMARY OF THE INVENTION

The inventor has developed an oral pharmaceutical composition and found that the oral pharmaceutical composition comprising essential oils exhibits surprising effects on treating and/or preventing diseases.

According to the present invention, there is provided an oral pharmaceutical composition containing:
- an essential oil portion comprising an essential oil derived from a plant, wherein the plant's genus is selected from the group consisting of *Matricaria, Citrus, Origanum, Rosmarinus, Juniperus* and *Lanvandula* and
- a carrier oil portion having a fatty acid.

Particularly, the invention provides an oral pharmaceutical composition comprising an essential oil portion and a carrier oil portion, wherein the essential oil portion consists essentially of
- from about 20 vol.-% to about 50 vol.-% of the essential oil derived from *Citrus bergamia* based on the total volume of the essential oil portion;
- from about 20 vol.-% to about 50 vol.-% of the essential oil derived from *Matricaria chamomilla* based on the total volume of the essential oil portion;
- from about 10 vol.-% to about 30 vol.-% of the essential oil derived from *Origanum vulgare* based on the total volume of the essential oil portion;
- from about 5% to about 20 vol.-% of the essential oil derived from *Juniperus communis* based on the total volume of the essential oil portion; and
- from about 5% to about 20 vol.-% of the essential oil derived from *Rosmarinus officinalis* based on the total volume of the essential oil portion; and
wherein a carrier oil portion comprises a fatty acid.

The oral pharmaceutical composition of the present invention has proved to be effective for treating and/or preventing a disease. The oral pharmaceutical composition of the present invention can be used in the preparation of a medicament for treatment or prevention of a disease. Without intending to be limited by theory, it is believed that in particular, the oral pharmaceutical composition is capable of reducing the levels of inflammatory factors such as interferon-gamma and tumor necrosis factor alpha, and increasing the anti-inflammatory factors such as interleukin-10. Also, it is believed that the oral pharmaceutical composition may improve the function of the organs, for example by maintaining alanine aminotransferase and aspartate aminotransferase levels in blood for exhibiting protective effects on liver.

The present invention further relates to a use of said oral pharmaceutical composition for treating and/or preventing a disease.

Additionally, the present invention relates to a food supplement composition containing an essential oil portion and a carrier oil portion, wherein the essential oil portion consists essentially of
- from about 20 vol.-% to about 50 vol.-% of the essential oil derived from *Citrus bergamia* based on the total volume of the essential oil portion;
- from about 20 vol.-% to about 50 vol.-% of the essential oil derived from *Matricaria chamomilla* based on the total volume of the essential oil portion;
- from about 10 vol.-% to about 30 vol.-% of the essential oil derived from *Origanum vulgare* based on the total volume of the essential oil portion;
- from about 5% to about 20 vol.-% of the essential oil derived from *Juniperus communis* based on the total volume of the essential oil portion; and
- from about 5% to about 20 vol.-% of the essential oil derived from *Rosmarinus officinalis* based on the total volume of the essential oil portion; and
wherein a carrier oil portion comprises a fatty acid.

The food supplement composition preferably includes essential fatty acids that a subject required from the food source. The food supplement composition of the present invention allows a subject to maintain a balanced diet by consuming the food supplement daily, weekly or monthly depending on the condition of the subject.

### BRIEF DESCRIPTION OF DRAWINGS

Figure 1 shows the mouse liver tissues after HE staining, in which the concanavalin A-induced mice were administered with an oral pharmaceutical composition according to the present invention, wherein Figure 1A is a mouse liver tissue from the normal control group,
Figure 1B is a mouse liver tissue from the model group, and Figure 1C to 1E are mouse liver tissues administered with different concentrations of the composition A of the present invention.
Figure 2 shows the expressions of CYP5A1, CYP2E1 and CYP3A in the mouse liver tissue after the concanavalin A-induced mice were administered with an oral pharmaceutical composition according to the present invention.

### DETAILED DESCRIPTION OF THE INVENTION

The present invention provides an oral pharmaceutical composition containing
- an essential oil portion containing an essential oil derived from a plant, wherein the plant's genus is selected from the group consisting of *Matricaria, Citrus, Origanum, Rosmarinus, Juniperus,* and *Lanvandula;* and
- a carrier oil portion having a fatty acid.

The term "oral pharmaceutical composition" of the present invention refers to a composition that is suitable for therapeutically and/or prophylactically treating or preventing a disease by administering to a subject orally, i.e. through an oral administration. Accordingly, the oral pharmaceutical composition of the present invention may be in the form of a dispersion, an emulsion, a suspension, a syrup, a tablet, a capsule, a hard or soft gelatin capsule, a pill, a pellet, a powder, a granule and the like. In a preferred embodiment, the oral pharmaceutical composition is in the form of an dispersion, an emulsion, a suspension, a hard or soft gelatin capsule, and/or a compressed tablet.

The oral pharmaceutical composition may include a pharmaceutically acceptable excipient or vehicle suitable for administration to a subject. In an embodiment herein, the subject is a mammal such as a rodent or human. Preferably, the subject is human. The pharmaceutically acceptable excipient includes a liquid or solid filler, a lubricant, a diluent, a solvent or an encapsulating material, for carrying and delivering active ingredients of the oral pharmaceutical composition to an organ or a part of the body of the subject to exert the therapeutic effect.

The oral pharmaceutical composition of the present invention contains an essential oil portion and a carrier oil portion. The amount of the essential oil portion is from about 0.1 vol.-% to about 20 vol.-% based on the total volume of the oral pharmaceutical composition. In one embodiment, the essential oil portion is from about 0.2 vol.-% to about 15 vol.-%; or from about 0.3 vol.-% to about 10 vol.-%; or from about 0.4 vol.-% to about 5 vol.-%; or from about 0.5 vol.-% to about 3 vol.-% based on the total volume of the oral pharmaceutical composition. In an embodiment herein, the amount of the essential oil portion is about 1 vol.-% based on the total volume of the oral pharmaceutical composition.

Moreover, the amount of the carrier oil portion is from about 80 vol.-% to about 99.9 vol.-% based on the total volume of the oral pharmaceutical composition. In one embodiment, the carrier oil portion is from about 85 vol.-% to about 99.8 vol.-%; or from about 90 vol.-% to about 99.7 vol.-%; or from about 95 vol.-% to about 99.6 vol.-%; or from about 97 vol.-% to about 99.5 vol.-% based on the total volume of the oral pharmaceutical composition. In an embodiment, the amount of the carrier oil portion is about 99 vol.-% based on the total volume of the oral pharmaceutical composition.

The term "essential oil", also known as ethereal oil and volatile oil, as used herein refers to a concentrated hydrophobic liquid containing volatile aroma compounds. The essential oil is generally obtained from a plant material by some form of extraction such as distillation, solvent extraction and cold pressing to isolate the volatile compounds from the plant material. Preferably, the plant material is non-transgenic. In an embodiment herein, the essential oil is in the form of an extract. The skilled person in the art is aware of suitable methods for obtaining essential oils from the plant material. In some embodiments, the essential oils of varying grades and purities are easily and also commercially available from vendors and companies around the world. The term "essential" in this particular phrase refers to "an essence" of the plant. It is appreciated that the skilled person would not confuse this term with "essential fatty acid" which refers to fatty acids humans or animals must require from the food source for health, i.e. for a balanced diet.

The essential oil portion of the present disclosure includes an essential oil derived from a plant, wherein the plant's genus is selected from the group consisting of *Matricaria, Citrus, Origanum, Rosmarinus, Juniperus,* and *Lanvandula.* In embodiments, the essential oil portion includes an essential oil derived from *Matricaria chamomilla, Citrus bergamia, Origanum vulgare, Juniperus communis* or *Rosmarinus officinalis.* In an embodiment, the essential oil portion includes an essential oil derived from *Matricaria chamomilla* and an essential oil derived from *Citrus bergamia.* In a further embodiment, the essential oil may further include an essential oil derived from *Origanum vulgare*; still further, the essential oil may additionally include an essential oil derived from *Juniperus communis* and an essential oil derived from *Rosmarinus officinalis.*

The essential oil portion consists essentially of
- from about 20 vol.-% to about 50 vol.-% of the essential oil derived from *Citrus bergamia* based on the total volume of the essential oil portion;
- from about 20 vol.-% to about 50 vol.-% of the essential oil derived from *Matricaria chamomilla* based on the total volume of the essential oil portion;
- from about 10 vol.-% to about 30 vol.-% of the essential oil derived from *Origanum vulgare* based on the total volume of the essential oil portion;
- from about 5% to about 20 vol.-% of the essential oil derived from *Juniperus communis* based on the total volume of the essential oil portion; and
- from about 5% to about 20 vol.-% of the essential oil derived from *Rosmarinus officinalis* based on the total volume of the essential oil portion.

The essential oil portion contains a range of terpenes and terpenoids, in particular monoterpene compounds and their derivatives. In embodiments, the essential oil portion includes a compound selected from the group consisting of limonene, 1,8-cineole and carvacrol. Limonene is a monoterpene having the following formula (I). 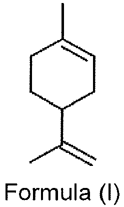 1,8-cineole is a monoterpenoid having the following formula (II) 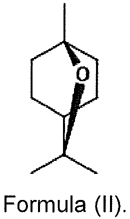 Carvacrol is a monoterpenoid phenol having the following formula (III)

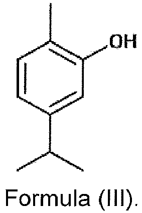

In one embodiment, the oral pharmaceutical composition includes at least 5% of limonene, at least 5% of 1,8-cineole and at least 5% of carvacrol based on the total volume of the oral pharmaceutical composition. In a further embodiment, the amount of each of limonene, 1,8-cineole and carvacrol in the oral pharmaceutical composition is from about 5% to 15%; or from about 8% to 10%, respectively, based on the total volume of the oral pharmaceutical composition. Accordingly, without intended to be limited by the theory, it is believed that the oral pharmaceutical composition exhibits protection effects on the organs of the subject against undesirable oxidation.

The term "carrier oil", also known as base oil, of the present invention refers to an oil-based liquid that used to dilute the essential oils to have an appropriate amount of essential oils in the oral pharmaceutical composition for exhibiting the desired effects. The carrier oil may also act as an acceptable agent in the composition. The carrier oil generally does not include concentrated aroma compounds. The carrier oil may be derived from a plant material or an animal material. In embodiments herein, the carrier oil is in the form of an extract, in particular an oil-based extract containing a vegetable fatty acid. The term "fatty acid" as used herein refers to a carboxylic acid having a long aliphatic chain. The fatty acid refers to both saturated fatty acids and unsaturated fatty acids. For "a vegetable fatty acid", it refers to the fatty acid derived from a plant material.

The vegetable fatty acids are derived from a plant, and wherein the plant's genus is selected from the group consisting of *Cannabis, Sesamum, Prunus dulcis* and *Rosmarinus.* In one embodiment, the vegetable fatty acids are derived from at least one species of *Cannabis sativa* L., *Cannabis indica* Lam., *Cannabis ruderalis* Janisch, *Sesamum indicum* L., *Prunus dulcis* (Mill.) D. A. Webb, and *Rosmarinus officinalis.* The skilled person is aware of suitable method of obtaining carrier oil from the plant material. Carrier oil of varying grades and purities is easily and also commercially available from vendors and companies around the world. For example, the vegetable fatty acids may be derived from commercially available hemps, sesames, sweet almonds or rosemary flowers.

In a preferred embodiment, the carrier oil portion of the oral pharmaceutical composition includes one or more oil-based extract derived from one or more plant materials. The plant material is selected from the group consisting of hemps, sesames and sweet almonds. In one embodiment, the carrier oil portion includes three oil-based extracts derived from hemps, sesames and sweet almonds, i.e. the carrier oil portion includes hemp oil, sesame oil and sweet almond oil. The amount of the hemp oil is from about 5 vol.-% to about 35 vol.-%; or from about 10 vol.-% to about 30 vol.-%; or from about 15 vol.-% to about 25 vol.-%; or about 20 vol.-% based on the total volume of the carrier oil portion. The amount of the sesame oil is from about 15 vol.-% to about 45 vol.-%; or from about 20 vol.-% to about 40 vol.-%; or from about 25 vol.-% to about 35 vol.-%; or about 30 vol.-% based on the total volume of the carrier oil portion. The amount of the sweet almond oil is from about 35 vol.-% to about 65 vol.-%; or from about 40 vol.-% to about 60 vol.-%; or from about 45 vol.-% to about 55 vol.-%; or about 50 vol.-% based on the total volume of the carrier oil portion. In particular, the carrier oil portion includes about 20 vol.-% hemp oil, about 30 vol.-% sesame oil and about 50 vol.-% sweet almond oil.

In an embodiment herein, the carrier oil portion of the present invention includes at least two vegetable fatty acids selected from the group consisting of oleic acid, vaccenic acid, linoleic acid, α-linolenic acid and γ-linolenic acid. In a further embodiment, the carrier oil portion includes all of oleic acid, vaccenic acid, linoleic acid, α-linolenic acid and γ-linolenic acid. In another embodiment, the oral pharmaceutical composition contains vaccenic acid in an amount of at least 0.5 wt.-%; or from about 0.5 wt.-% to 10 wt.-%; or from about 0.8 wt.-% to 5 wt.-%; or from about 1 wt.-% to 3 wt.-% based on the total amount of fatty acids in the oral pharmaceutical composition. In one embodiment, the oral pharmaceutical composition includes oleic acid in an amount of from about 20 wt.-% to 60 wt.-%; or from about 30 wt.-% to 55 wt.-%; or from about 40 wt.-% to 50 wt.-% based on the total amount of fatty acids in the oral pharmaceutical composition. In another embodiment, the oral pharmaceutical composition includes linoleic acid in an amount of from about 20 wt.-% to 60 wt.-%; or from about 35 wt.-% to 55 wt.-%; or from about 30 wt.-% to 40 wt.-% based on the total amount of fatty acids in the oral pharmaceutical composition. In a further embodiment, the oral pharmaceutical composition includes α-linolenic acid in an amount of at least 0.5 wt.-%; or from about 0.5 wt.-% to 10 wt.-%; or from about 0.8 wt.-% to 5 wt.-%; or from about 1 wt.-% to 4 wt.-% based on the total amount of fatty acids in the oral pharmaceutical composition. In another embodiment, the oral pharmaceutical composition includes γ-linolenic acid in an amount of at least 0.5 wt.-%; or from about 0.5 wt.-% to 10 wt.-%; or from about 0.8 wt.-% to 5 wt.-%; or from about 1 wt.-% to 3 wt.-% based on the total amount of fatty acids in the oral pharmaceutical composition.

Without intending to be limited by theory, it is believed that the vegetable fatty acids in the oral pharmaceutical composition may help inhibit the release of arachidonic acid, exhibit anti-coagulate effect, improve the blood vessel elasticity, lower the level of the bad cholesterol, i.e. low-density lipoproteins, in blood, and/or inhibit the release of inflammatory factors and improve the insulin's action.

In embodiments of the present invention, the oral pharmaceutical composition may additionally include a polymer to encapsulate the essential oil portion and the carrier oil portion in the polymer. In an embodiment herein, the essential oil portion and the carrier oil portion may be coated with a coating film consisting essentially of the polymer. Preferably, the polymer is a polysaccharide such as sodium alginate. In another embodiment, the polymer may be polylactic acid, poly lactic-co-glycolic acid or the combination thereof.

For ease of storage and administration, the oral pharmaceutical composition may be encapsulated to form microcapsules. It allows the oral pharmaceutical composition to be stored at a relatively more stable condition and provides an alternative dosage form for a user to intake said composition. Such microcapsules allows for a more user-friendly approach for oral administration. In one embodiment, a micro-encapsulating system may be applied to form the microcapsules. The micro-encapsulating system preferably includes a jet-flow microencapsulation technique.

In an embodiment, the method of encapsulating the essential oil portion and the carrier oil portion to form microcapsules includes the following steps of:
- mixing the essential oil portion and the carrier oil portion thoroughly with a mixture of a polymer and water, preferably said a polymer comprises sodium alginate, to form a polymeric solution;
- introducing the polymeric solution into a gelifying solution by using a syringe with a predetermined flow rate to form microcapsules, preferably the gelifying solution comprises calcium chloride or calcium salts;
- curing the microcapsules in the gelifying solution with a constant magnetic stirring for a predetermined period of time;
- filtering the cured microcapsules;
- re-suspending the filtered microcapsules to form a suspension for lyophilization; and
- collecting the resultant microcapsules.

It will be appreciated by the person skilled in the art that other suitable methods for preparing microcapsules may be applied to the present invention.

Moreover, the present invention also pertains to treating and/or preventing a disease in a subject by administering an effective amount of an oral pharmaceutical composition, as described above, to the subject.

The expression "effective amount" generally denotes an amount sufficient to produce therapeutically desirable results, wherein the exact nature of the result varies depending on the specific disease which is treated. When the disease is accompanied by inflammation, the result is usually a suppression or decrease of the expression or functional activity of inflammatory factors. The results may also be an increase of the expression or functional activity of anti-inflammatory factors. It will be appreciated by a physician or veterinarian having ordinary skill in the art to determine and prescribe the effective amount of the oral pharmaceutical composition of the present invention. The physician or veterinarian will also capable of adjusting the effective amount of the oral pharmaceutical composition according to the severity of the condition to be alleviated.

In an embodiment, where the subject is human, the oral pharmaceutical composition is administered to the subject in an amount of from about 0.04 ml/kg to about 0.4 ml/kg; or from about 0.05 ml/kg to about 0.3 ml/kg based on the total body weight of the subject. In particular, the oral pharmaceutical composition is administered to the subject in an amount of about 0.055 ml/kg, 0.11 ml/kg or 0.22 ml/kg daily depending on the severity of the condition to be alleviated. The oral pharmaceutical composition may be administered in the form of liquid.

In another embodiment, where the subject is a rodent, the oral pharmaceutical composition is administered to the subject in an amount of from about 0.5 ml/kg to about 2.0 ml/kg based on the total body weight of the subject. In particular, the oral pharmaceutical composition is administered to the subject in an amount of about 0.5 ml/kg, about 1 ml/kg or about 2 ml/kg daily depending on the severity of the condition to be alleviated. The oral pharmaceutical composition may be administered in the form of liquid.

Without intending to be limited by theory, it is believed that the composition of the present invention is effective for treating and/or preventing inflammatory disease such as autoimmune disease, liver disease and cardiovascular disease which may or may not be companied by an inflammatory response. In embodiments herein, the composition is administered to treat and/or prevent a liver disease or a cardiovascular disease.

The liver disease, also referred as hepatic disease, covers all potential disorders of the liver in which the liver fails to function properly. The liver disease may be selected from the group consisting of hepatitis, alcoholic liver disease, fatty liver disease, hereditary disease, fascilasis, cirrhosis, liver cancer and a combination thereof. In some embodiments, the oral pharmaceutical composition is used for treating and/or preventing hepatitis, alcoholic liver disease or fatty liver disease in the subject.

The cardiovascular disease covers all potential disorders of the heart and blood vessels in which the heart and blood vessels fail to function properly. The cardiovascular disease may be selected from the group consisting of atherosclerosis, restenosis, coronary arterial disease, peripheral arterial disease, aneurysm, thrombosis, embolism, myocardial infarction, ischemia and a combination thereof. In embodiments herein, the oral pharmaceutical composition is used for treating and preventing atherosclerosis, coronary arterial disease or peripheral arterial disease in the subject.

In other embodiments, the composition is administered to treat a gastrointestinal disease, in particular a gastrointestinal disease accompanied by an inflammation such as gastritis, gastroenteritis, gastric ulceration, peptic ulcers, enterocolitis, diarrhorea and colitis.

The oral pharmaceutical composition as used herein may also be combined with one or more other therapeutic agent suitable for particular indication being treated. The therapeutic agent is present with an effective amount to achieve the desired therapeutic effect.

The use as described herein provides an alternative way for a physician or veterinarian to treat and/or prevent a subject from suffering certain diseases or disorders. In particular, the use of the present invention is efficacious in treating and/or preventing a liver disease and a cardiovascular disease in a subject without exerting detrimental effects on the liver. The method may also allow for a generally reduced risk for severe side effects.

In addition, the present invention further relates to a food supplement composition. The food supplement composition contains an essential oil portion and a carrier oil portion as described in the claims.

The term "food supplement" as used in the present invention refers to a composition intends to provide nutrients to a subject to maintain a balanced diet. The terms essential oil and carrier oil are defined elsewhere herein.

In one embodiment, the food supplement composition includes from about 0.1 vol.-% to about 5 vol.-%; or from about 0.2 vol.-% to about 2 vol.-%; or from about 0.3 vol.-% to about 1 vol.-% of the essential oil portion based on the total volume of the food supplement composition. Also, the food supplement composition includes from about 95 vol.-% to about 99.9 vol.-%; or from about 98 vol.-% to about 99.8 vol.-%; or from about 99 vol.-% to about 99.7 vol.-% of the carrier oil portion based on the total volume of the food supplement composition. In particular, the food supplement composition includes about 1 vol.-% of the essential oil portion and about 99 vol.-% of the carrier oil portion.

Further, the essential oil portion consists essentially of
- from about 20 vol.-% to about 50 vol.-% of the essential oil derived from *Citrus bergamia* based on the total volume of the essential oil portion;
- from about 20 vol.-% to about 50 vol.-% of the essential oil derived from *Matricaria chamomilla* based on the total volume of the essential oil portion;
- from about 10 vol.-% to about 30 vol.-% of the essential oil derived from *Origanum vulgare* based on the total volume of the essential oil portion;
- from about 5% to about 20 vol.-% of the essential oil derived from *Juniperus communis* based on the total volume of the essential oil portion; and
- from about 5% to about 20 vol.-% of the essential oil derived from *Rosmarinus officinalis* based on the total volume of the essential oil portion.

The essential oil portion of the food supplement composition also comprises a compound selected from the group consisting of limonene, 1,8-cineole and carvacrol. In particular, the food supplement composition comprises at least 5% of limonene, at least 5% of 1,8-cineole and at least 5% of carvacrol based on the volume of the food supplement composition.

Preferably, the food supplement composition includes vegetable fatty acids derived from at least one species of *Cannabis sativa* L., *Cannabis indica* Lam., *Cannabis ruderalis* Janisch, *Sesamum indicum* L., *Prunus dulcis* (Mill.) D. A. Webb, or *Rosmarinus officinalis.* In an embodiment, the food supplement composition includes one or more oil-based extract derived from a plant material selected from the group consisting of hemps, sesames and sweet almonds, i.e. the carrier oil portion includes hemp oil, sesame oil and sweet almond oil.

The amount of the hemp oil is from about 5 vol.-% to about 35 vol.-%; or from about 10 vol.-% to about 30 vol.-%; or from about 15 vol.-% to about 25 vol.-%; or about 20 vol.-% based on the total volume of the carrier oil portion. The amount of the sesame oil is from about 15 vol.-% to about 45 vol.-%; or from about 20 vol.-% to about 40 vol.-%; or from about 25 vol.-% to about 35 vol.-%; or about 30 vol.-% based on the total volume of the carrier oil portion. The amount of the sweet almond oil is from about 35 vol.-% to about 65 vol.-%; or from about 40 vol.-% to about 60 vol.-%; or from about 45 vol.-% to about 55 vol.-%; or about 50 vol.-% based on the total volume of the carrier oil portion. In particular, the carrier oil portion includes about 20 vol.-% hemp oil, about 30 vol.-% sesame oil and about 50 vol.-% sweet almond oil.

The carrier oil portion comprises at least two fatty acids selected from the group consisting of oleic acid, vaccenic acid, linoleic acid, α-linolenic acid, and γ-linolenic acid. In particular, the food supplement composition includes vegetable fatty acids of oleic acid, vaccenic acid, linoleic acid, α-linolenic acid and γ-linolenic acid.

In one embodiment, the food supplement composition includes:
- from about 0.5 wt.-% to 10 wt.-%; or from about 0.8 wt.-% to 5 wt.-%; or from about 1 wt.-% to 3 wt.-% of vaccenic acid based on the total amount of fatty acids in the food supplement composition;
- from about 20 wt.-% to 60 wt.-%; or from about 30 wt.-% to 55 wt.-%; or from about 40 wt.-% to 50 wt.-% of oleic acid based on the total amount of fatty acids in the food supplement composition;
- from about 20 wt.-% to 60 wt.-%; or from about 35 wt.-% to 55 wt.-%; or from about 30 wt.-% to 40 wt.-% of linoleic acid based on the total amount of fatty acids in the food supplement composition;
- from about 0.5 wt.-% to 10 wt.-%; or from about 0.8 wt.-% to 5 wt.-%; or from about 1 wt.-% to 4 wt.-% of α-linolenic acid based on the total amount of fatty acids in the food supplement composition; and
- from about 0.5 wt.-% to 10 wt.-%; or from about 0.8 wt.-% to 5 wt.-%; or from about 1 wt.-% to 3 wt.-% of γ-linolenic acid based on the total amount of fatty acids in the food supplement composition.

Without intending to be limited by theory, it is believed that the food supplement composition of the present invention provides sufficient nutrients to a subject who lacks certain nutrients. Said nutrients the subject requires may include essential fatty acids, e.g. oleic acid, linoleic acid and α-linolenic acid. Therefore, the present invention provides a food supplement that a subject can consume, for example intake daily, to maintain a balanced diet for health. The food supplement composition may be taken once or twice daily, weekly or monthly depending on the necessity in combination with usual daily food source.

In an embodiment herein, where the subject is human, the subject may intake the food supplement composition in an amount of from about 0.01 ml/kg to 0.1 ml/kg daily; or from about 0.02 ml/kg to 0.08 ml/kg daily; or from about 0.03 ml/kg to 0.06 ml/kg daily based on the total body weight of the subject. In particular, the subject may intake the food supplement composition in an amount of about 0.05 mg/kg daily.

In another embodiment, the food supplement may further include a polymer to encapsulate the essential oil portion and the carrier oil portion. As described elsewhere herein, the polymer may form a coating film to coat the essential oil portion and the carrier oil portion. In one embodiment, the food supplement composition may be in the form of microcapsules such that the food supplement composition can be stored at a relative stable condition and provides a more user-friendly form for a user to intake the food supplement composition. In particular, the microcapsules may be prepared according to the similar method as described above for the oral pharmaceutical composition.

The examples set out below further illustrate the present invention. The preferred embodiments described above as well as examples given below represent preferred or exemplary embodiments and a skilled person will understand that the reference to those embodiments or examples is not intended to be limiting.

### EXAMPLE 1 (comparative)

### Preparation of oral pharmaceutical composition

An oral pharmaceutical composition was prepared with the following ingredients:
- 2.5 ml *Citrus bergamia* essential oil;
- 2.5 ml *Matricaria chamomilla* essential oil;
- 1.9 ml *Origanum vulgare* essential oil;
- 1.25 ml *Juniperus communis* essential oil;
- 1.25 ml *Rosmarinus officinalis* essential oil;
- 0.6 ml Lavender essential oil;
- 200 ml hemp oil;
- 300 ml sesame oil; and
- 490 ml sweet almond oil.

The above ingredients were obtained from commercial suppliers. In particular, the hemp oil was derived from non-transgenic plants of *Cannabis sativa* L., *Cannabis indica* Lam. and/or *Cannabis ruderalis.* The ingredients were measured and mixed thoroughly by using a vortex mixer to obtain a homogenous oil solution.

In other examples, additional carrier oils derived from other plant material, such as evening primrose, may also be added to the composition to make up to the total volume of the carrier oil portion.

### EXAMPLE 2

### Active Fatty Acid and soluble fatty substances in the composition

A fatty acid testing was conducted to confirm the active fatty acid components in the oral pharmaceutical composition as obtained form Example 1. The fatty acid testing was conducted by an ISO accredited laboratory with standard testing conditions and procedures. Table 1 shows the fatty acid contents in the oral pharmaceutical composition, in particular said composition includes 1.5% vaccenic acid, 42% oleic acid, 34% linoleic acid, 3.5% α-linoleic acid, 1.7% γ-linoleic acid and other fatty acid components.

**Table 1A. Fatty acids and fat soluble substances in the oral pharmaceutical composition**

| **Component(s)** | **Amount based on the total amount of the fatty acids in the composition** |
|---|---|
| Palmitic acid | 6.2% |
| Stearic acid | 2.7% |
| Oleic acid | 42% |
| Vaccenic acid | 1.5% |
| Linoleic acid | 34% |
| α-linoleic acid | 3.5% |
| γ-linoleic acid | 1.7% |
| Vitamin A (Retinol) | <100 IU/kg |
| Vitamine E (α-tocopherol) | 193 ppm |

The fatty acids are primarily derived from the carrier oils. The high values of oleic acid and linoleic acid are advantageous in that they help to decrease the level of low-density lipoprotein cholesterol in a subject. The presence of these fatty acids in the composition further maintain the health of the subject by providing sufficient nutrients to a subject who lacks certain essential nutrients.

### EXAMPLE 3

### Determination of limonene, 1,8-cineole and carvarol

The oral pharmaceutical composition was further subjected to a chromatographic analysis to determine the concentrations of limonene, 1,8-cineole, carvarol and other components in the composition. In particular, a validated gas chromatography (GC) was conducted using standard conditions in the art. The oral pharmaceutical composition and standard solutions were injected to the GC system for testing. The GC results reveal 53 components. Among the 53 components, 10 of them have an amount of more than 3 vol.-% based on the total volume of the oral pharmaceutical composition. Table 1B shows a part of the GC results analysis.

**Table 1B. GC results of 10 components in the oral pharmaceutical composition.**

| **Component(s)** | **Amount based on the total volume of the oral pharmaceutical composition** |
|---|---|
| Alpha-pinene | 5.8% |
| Limonene | 9.66% |
| 1,8-cineole | 9.34% |
| Linalool | 4.60% |
| Neral | 4.74% |
| Linalyl acetate | 6.23% |
| Geranial | 7.17% |
| Carvacrol | 8.05% |
| Eugenol | 5.63% |
| Trans-beta-femesene | 4.41% |

Specifically, the results confirm that there are 9.66% limonene, 9.34 % 1,8-cineole, and 8.05% carvarol based on the total volume of the oral pharmaceutical composition.

### EXAMPLE 4

### Effects on leukotriene level and ALT level in an animal model suffering from an induced acute liver injury

The level of alanine aminotransferase (ALT), which formerly called serum glutamate-pyruvate transaminase, is generally an indication of whether the liver of the tested subject is damaged/ malfunctioned or not. ALT, i.e. a transaminase enzyme, is most commonly found in the liver. When the liver is injured, the liver releases ALT to the bloodstream. To confirm whether the liver is injured or not, a further factor aspartate transaminase (AST) level is usually measured to form a ratio with the detected value of ALT. If the detected level of AST is significantly lower than that of ALT, the liver is considered damaged. In turn, if ASTALT ratio is larger than 2:1, it may suggest that the subject is suffering from an alcoholic liver disease.

To determine the effects of the oral pharmaceutical composition on leukotriene level and ALT level, an animal model was set up by using P. acnes-lipopolysaccharide (LPS) to induce an acute liver injury. Seven-week old male (18-22g body weight) Kunming mice (KM mice) were used. Heat-killed P. acnes was injected on day 2 of oral gavage treatment through the tail vein with a dosage of 20 mg/kg/mouse. After 5 days, LPS (5 µg/ kg /mouse) was injected intravenously.

5 h after the LPS injection, the mice were sacrificed for analyzing liver injury. Blood samples were taken from the heart of the mice under anesthesia with diethyl ether. The blood samples were collected in a tube containing 2% sodium heparin. The tubes were centrifuged at 1000 g r/ min for 15 min and the supernatant was collected as testing samples. All testing samples were stored at -20 °C before performing subsequent assays.

The mice were randomly divided into 5 groups and each group had 10 mice. The 5 groups are control group, liver injured model group, and 3 treatment groups. The 3 treatment groups are further as follows:
- High dosage group, hereinafter denoted with "Ah": a dose of 0.04ml/day/20g of the oral pharmaceutical composition was administered to the mice orally;
- Moderate dosage group, hereinafter denoted with "Am": a dose of 0.02ml/day/20g of the oral pharmaceutical composition was administered to the mice orally; and
- Low dosage group, hereinafter denoted with "Al": a dose of 0.01ml/day/20g of the oral pharmaceutical composition was administered to the mice orally.

The total volume for single oral gavage treatment for each of the mice in the groups was 0.5 ml.

Specifically, mice were pretreated orally (oral gavage) with vehicle (distilled water for the control group) or indicated dosages of composition once a day for 7 consecutive days. These oral administrations were conducted one day before establishing the model. The last oral administration was performed 30 min before the LPS injection. Blood samples were collected from the posterior vena cava, centrifuged and stored for biochemical analysis. The biochemical analysis was further conducted to determine the level of leukotriene B4 and C4, as well as ALT in the testing samples.

For the biochemical analysis. ELISA kits were used and standard curves were plotted using standard solutions. Table 2 to 4 show the results obtained from the ELISA tests. First of all, the disease model of liver injury was successfully established in view of the significant increase of the ALT levels, and leukotriene B4 and C4 levels.

From Table 2, compared the results in the model group, the oral pharmaceutical composition exhibited a dose dependent reduction on ALT level. Table 3 and Table 4 reveal that the oral pharmaceutical composition is capable of lowering the leukotriene B4 and C4 levels. Accordingly, the oral pharmaceutical composition is proved to be effective for preventing an acute liver injury.

**Table 2. Effect of different doses of the oral pharmaceutical composition on ALT level**

| **Group** | **ALT (mU/mL)** | **vs. Control (%)** | **vs. Model (%)** |
|---|---|---|---|
| Control | 1.444±0.499 | 100.0 | 42.7 |
| Model | 3.382±1.137## | 234.2 | 100.0 |
| Ah | 1.971:1:0.861* | 136.5 | 58.3 |
| Am | 1.62010.691** | 112.2 | 47.9 |
| Al | 2.982±1.068 | 206.5 | 88.2 |

| | | | |
|---|---|---|---|
| ##P < 0.01 vs. control group; *P < 0.05, **P < 0.01 vs. model group. | | | |

**Table 3. Effect of different doses of the oral pharmaceutical composition on LTB4 levels**

| **Group** | **LTB4 (ng/mL)** | **vs. Control (%)** | **vs. Model (%)** |
|---|---|---|---|
| Control | 0.275±0.046 | 100.0 | 51.6 |
| Model | 0.532±0.655## | 193.5 | 100.0 |
| Ah | 0.266±0.957** | 136.5 | 50.0 |
| Am | 0.315±0.976** | 96.7 | 59.2 |
| Al | 0.347±0.156** | 126.2 | 65.2 |

| | | | |
|---|---|---|---|
| ##P < 0.01 vs. control group; *P < 0.05, **P < 0.01 vs. model group. | | | |

**Table 4. Effect of different doses of the oral pharmaceutical composition on LTC4 levels**

| **Group** | **LTC4 (ng/mL)** | **vs. Control (%)** | **vs. Model (%)** |
|---|---|---|---|
| Control | 1.380±0.221 | 100.0 | 75.9 |
| Model | 1.817±0.102## | 131.7 | 100.0 |
| Ah | 1.472±0.094** | 106.7 | 81.0 |
| Am | 1.531±0.215* | 110.9 | 84.3 |
| Al | 1.526±0.198* | 110.6 | 84.0 |

| | | | |
|---|---|---|---|
| ##P < 0.01 vs. control group; *P < 0.05, **P < 0.01 vs. model group. | | | |

### EXAMPLE 5

### Effects on cytokines level in animal models

Further animal tests were conducted to determine the effects of the oral pharmaceutical composition on the levels of cytokines, in particular in three more animal models. Levels of alanine aminotransferase (ALT), interleukin-10 (IL-10), interferon-γ (IFN- γ), tumor necrosis factor-a (TNF- α), total cholesterol (TC), triglyceride (TG), and prostaglandin E1 (PGE1) were determined.

In this example, two sets of oral pharmaceutical compositions were applied.
- The oral pharmaceutical composition as obtained form Example 1, hereinafter denoted with "Composition A", while "Ah". "Am", and "Al" are also used to denote said composition with different dosages;
- A boiled composition prepared by heating the oral pharmaceutical composition obtained from Example 1 to 100°C for 5 min and cooling it to room temperature before administration, said boiled composition is hereinafter denoted with "Composition A100", while "A100h", "A100m" and "A100l" are also used to denote said composition with different dosages.

Similar to Example 4, the mice in high dosage groups (Ah and A100h) were orally administered a dose of 0.04ml/day/20g of the compositions as described above. The mice in in moderate dosage groups (Am and A100m) were orally administered a dose of 0.02ml/day/20g of the compositions as described above. The mice in in low dosage groups (Al and A100l) were orally administered a dose of 0.01ml/day/ 20g of the compositions as described above.

Notably, the high dosage, moderate dosage and low dosage as describe above are essentially equivalent to 0.22 ml/day/kg, 0.1 ml/day/kg, 0.055 ml/day/kg respectively for adults.

24 mice were randomly divided into 8 groups, namely a control group, a disease model group, a high dosage group of Composition A (Ah), a moderate dosage group of Composition A (Am), a low dosage group of Composition A (Al), a high dosage group of Composition A100 (A100h), a moderate dosage group of Composition A100 (A100m), and a low dosage group of Composition A100 (A100l),

SPSS software was used in statistical analysis to ensure no statistical discrepancy exists among the groups. In particular, SPSS13.0 was used to perform the statistical analysis. The experimental results are presented with mean ± SD values. P<0.05 is statistically significant.

### EXAMPLE 5A

### Effects on animal model having liver injury induced by CCl4

KM mice were pretreated orally with vehicle (saline) or indicated dosages of targeted compositions (e.g. low, moderate and high) once a day for 7 consecutive days. Mice were fasted for 16 hr and CCl4 (20% in peanut oil, 2 ml/kg) was administrated orally to induce a liver injury in the mice. After 48 hr, mice were sacrificed. Blood samples were collected. The plasma of the blood samples were collected by placing the blood samples at room temperature for 2 hr. The plasma samples were stored at -20°C before subsequent biochemical analysis.

ELISA kits were used and standard curves were plotted using standard solutions. Table 7 to 13 show the cytokines levels obtained from the ELISA tests. The results show that the Composition A can significantly improve the ALT, IL-10, IFN-γ, TNF-α and PEG1 levels in the CCl4-induced acute liver injury. Composition A with a high dosage and a moderate dosage can exhibit effects on lowering TG and TC levels.

For Composition A100, it can also significantly improve the ALT levels. The Composition A100 also reduces the IFN-γ and TNF-α levels but in a non-significant manner. However, it has no effect on modulating the IL-10, TG, TC and PGE1 levels.

Accordingly, the results prove that Composition A can exhibit protection effects on the liver. Composition A can thus protect a subject from suffering from an acute liver injury. It is also recommended that the oral pharmaceutical composition of the present invention should not be heated or boiled before administration. Furthermore, it is suggested not to intake the oral pharmaceutical composition with hot food so as to maintain the therapeutic effect of the composition. Without intending to be limited by theory, it is believed that the components in the Composition A altered in the heat treatment process, e.g. some active components changed or evaporated, and thus Composition A100 produced relatively mild or less effects when compared with the untreated Composition A.

**Table 7. Effect of different doses of the oral pharmaceutical compositions on ALT levels**

| **Group** | **ALT (mU/mL)** | **vs. Control (%)** | **vs. Model (%)** |
|---|---|---|---|
| Control | 1.437±0.074 | ------ | 18.53 |
| Model | 7.756±0.319 ## | 539.7 | ------ |
| Ah | 3.425±0.108 ** | 238.3 | 44.16 |
| Am | 3.086±0.203 ** | 214.8 | 39.79 |
| Al | 2.731±0.169 ** | 190.0 | 35.21 |
| A100h | 6.668±0.074 ** | 464.0 | 85.97 |
| A100m | 6.495±0.154 ** | 452.0 | 83.74 |
| A100l | 5.603±0.127 ** | 389.9 | 72.24 |

| | | | |
|---|---|---|---|
| ## P<0.01 vs. control group; * P <0.05, ** P< 0.01 vs. model group. | | | |

**Table 8. Effect of different doses of the oral pharmaceutical compositions on IL-10 levels**

| **Group** | **IL-10 (pg/mL)** | **vs. Control (%)** | **vs. Model (%)** |
|---|---|---|---|
| Control | 103.80±7.12 | ------ | 108.54 |
| Model | 95.63±5.82 | 92.13 | ------ |
| Ah | 112.69±3.15* | 108.56 | 117.84 |
| Am | 111.04±7.30* | 106.97 | 116.11 |
| Al | 111.15±1.29* | 107.08 | 116.23 |
| A100h | 98.06±1.94 | 94.47 | 102.54 |
| A100m | 99.50±6.79 | 95.86 | 104.05 |
| A100l | 102.06±3.43 | 98.32 | 106.72 |

| | | | |
|---|---|---|---|
| ## P<0.01 vs. control group; * P <0.05, ** P< 0.01 vs. model group. | | | |

**Table 9. Effect of different doses of the oral pharmaceutical compositions on IFN-γ levels**

| **Group** | **IFN-γ (pg/mL)** | **vs. Control (%)** | **vs. Model (%)** |
|---|---|---|---|
| Control | 32.95±2.49 | ------ | 40.39 |
| Model | 81.58±2.52## | 247.58 | ------ |
| Ah | 43.90±4.97** | 133.23 | 53.81 |
| Am | 62.34±3.27** | 189.19 | 76.42 |
| Al | 54.24±10.89** | 164.61 | 66.49 |
| A100h | 62.29±4.19** | 189.04 | 76.35 |
| A100m | 73.56±10.22 | 223.25 | 90.17 |
| A100l | 77.13±8.15 | 234.08 | 94.54 |

| | | | |
|---|---|---|---|
| ## P<0.01 vs. control group; * P <0.05, ** P< 0.01 vs. model group. | | | |

**Table 10. Effect of different doses of the oral pharmaceutical compositions on TNF-α levels**

| | | | |
|---|---|---|---|
| **Group** | **TNF-α (pg/mL)** | **vs. Control (%)** | **vs. Model (%)** |
| Control | 317.93±24.37 | ------ | 61.83 |
| Model | 514.16±21.94## | 161.72 | ------ |
| Ah | 379.77±25.43** | 119.45 | 73.86 |
| Am | 477.76±7.77* | 150.28 | 92.92 |
| Al | 502.12±14.62 | 157.94 | 97.66 |
| A100h | 467.09±8.33** | 146.92 | 90.84 |
| A100m | 484.53±25.08 | 152.40 | 94.23 |
| A100l | 480.48±23.90 | 151.13 | 93.45 |

| | | | |
|---|---|---|---|
| ## P<0.01 vs. control group; * P <0.05, ** P< 0.01 vs. model group. | | | |

**Table 11. Effect of different doses of the oral pharmaceutical compositions on TG levels**

| **Group** | **TG (mmol/L)** | **vs. Control (%)** | **vs. Model (%)** |
|---|---|---|---|
| Control | 1.34±0.10 | ------ | 79.25 |
| Model | 1.69±0.15## | 126.18 | ------ |
| Ah | 1.44±0.06** | 107.98 | 85.57 |
| Am | 1.38±0.10** | 103.49 | 82.02 |
| Al | 1.50±0.06* | 112.47 | 88.34 |
| A100h | 1.45±0.04 | 108.48 | 89.13 |
| A100m | 1.66±0.13 | 120.20 | 85.97 |
| A100l | 1.56±0.08 | 116.96 | 95.26 |

| | | | |
|---|---|---|---|
| ## P<0.01 vs. control group; * P <0.05, ** P< 0.01 vs. model group. | | | |

**Table 12. Effect of different doses of the oral pharmaceutical compositions on TC levels**

| **Group** | **TC (mmol/L)** | **vs. Control (%)** | **vs. Model (%)** |
|---|---|---|---|
| Control | 2.94±0.29 | ------ | 60.19 |
| Model | 4.89±0.30## | 166.32 | ------ |
| Ah | 3.63±0.42** | 123.47 | 74.23 |
| Am | 4.13±0.07** | 140.48 | 84.46 |
| Al | 4.32±0.19* | 146.94 | 88.34 |
| A100h | 4.93±0.07 | 167.80 | 100.89 |
| A100m | 4.77±0.56 | 162.47 | 97.68 |
| A100l | 5.08±0.11 | 172.90 | 103.95 |

| | | | |
|---|---|---|---|
| ## P<0.01 vs. control group; * P <0.05, ** P< 0.01 vs. model group. | | | |

**Table 13. Effect of different doses of the oral pharmaceutical compositions on PGE1 levels**

| **Group** | **PGE1 (pg/L))** | **vs. Control (%)** | **vs. Model (%)** |
|---|---|---|---|
| Control | 803.28±82.84 | ------ | 61.16 |
| Model | 1313.36±101.77## | 163.50 | ------ |
| Ah | 920.73±16.52** | 114.62 | 70.11 |
| Am | 1022.93±57.61** | 127.34 | 77.89 |
| Al | 1079.04±54.24** | 134.33 | 82.16 |
| A100h | 1324.86±66.37 | 164.93 | 100.87 |
| A100m | 1293.30±47.69 | 161.00 | 98.47 |
| A100l | 1342.67±75.40 | 167.15 | 102.23 |

| | | | |
|---|---|---|---|
| ## P<0.01 vs. control group; * P <0.05, ** P< 0.01 vs. model group. | | | |

### EXAMPLE 5B

### Effects on animal model having an autoimmune hepatitis induced by concanavalin A

KM mice were pretreated orally with vehicle (saline) or indicated dosages of targeted compositions (e.g. low, moderate and high), once a day for 7 consecutive days. The mice were fasted overnight and then subjected to intravenous injection through the tail vein with concanavalin A (Con A, Sigma Chimica, Milan, Italy) solution. Con A solution was prepared by a dilution with sterile PBS so as to form a dose of 20 mg/kg body weight. The model group was observed for histological signs of hepatitis within 8 h after administration. Blood samples were collected from the posterior vena cava. Plasma of the blood samples were collected and stored at -20 °C before subsequent biochemical analysis. In addition, the liver tissues were used in HE staining and Western blot.

ELISA kits were used and standard curves were plotted using standard solutions. Table 14 to 20 show the cytokines levels obtained from the ELISA tests. The results show that Composition A can significantly improve the levels of ALT, IL-10, IFN-γ, TNF-α and PEG1 in Con A induced-hepatitis. The composition has no observable effects on TG and TC levels. Regarding Composition A100, there are no significant improvements in cytokine level. It only shows a mild effect on improving the TNF-α level.

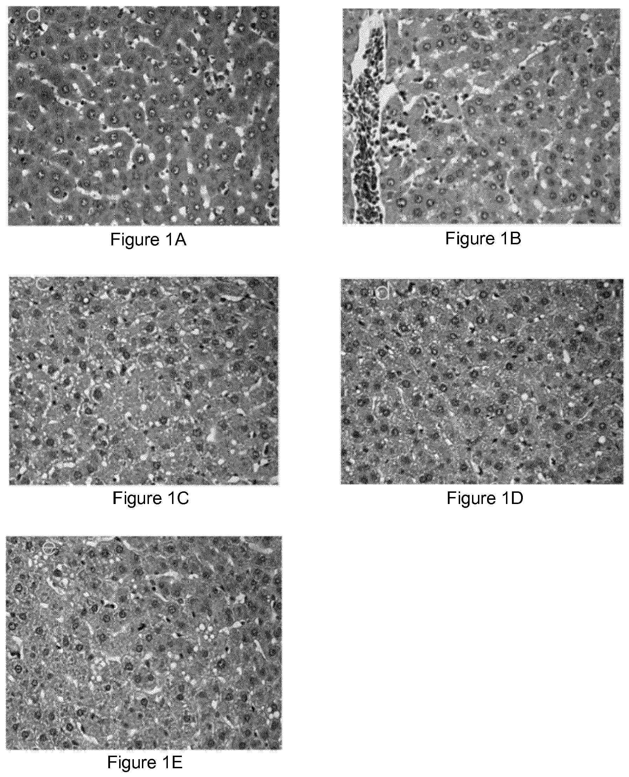

A part of the liver tissue was fixed by using neutral formalin and embedded in paraffin. After dewaxing, it was soaked with 100%, 95%, 70% alcohol gradient and rinsed briefly with distilled water. Then, hematoxylin staining and eosin solution treatment were carried out. Finally, pathological changes of the liver tissue were observed under an optical microscope. In the normal control group, there are no abnormalities in the mouse liver cells and hepatic sinus (see Figure 1A). In the model group, there are many apoptotic cells located in hepatic lobules or hepatic sinus in the liver tissue; the volume of apoptotic liver cells is smaller than that of that normal liver cells; the cells shrunk; vacuoles appear in the cytoplasm; and the nucleus and cytoplasm are condensed. There are many liver cell apoptotic bodies. The apoptotic bodies are round and different in size. Under HE staining, the cytoplasm of the corpuscles is eosinophilic, the central nucleus is deeply stained, the nucleus is condensed, and some nuclei are fragmented. The degree of liver cell necrosis is relatively mild, point-like, and focal necrosis. Most of the cytoplasm of the liver cells are swollen and loose, and some are balloon-like. There are Kupffer cell proliferation, red blood cell accumulation in the hepatic sinus, and fibrous tissue hyperplasia in the portal area (see Figure 1B). In the Composition A treatment group, there are reduced liver cell necrosis and apoptotic cells, and reduced inflammatory cell infiltration (see Fig. 1C-1E).

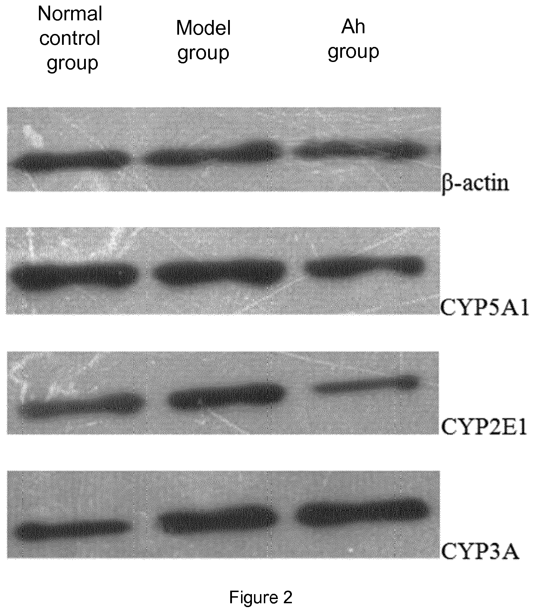

Another part of the liver tissue was processed by a homogenizer. The protein sample was extracted and denatured for storage at -20°C. 10% SDS-PAGE gel electrophoresis was conducted. After electrophoresis, the proteins were transferred onto a PVDF membrane (Bio-Rad). Skim milk was used for blocking overnight. The membrane was then incubated with a primary antibody - 2% bovine serum albumin (CYP5A1, CYP2E1, CYP3A) for 3 hours at room temperature. TBST was used for washing for 3 times with 10 minutes each time. A secondary antibody diluted with 2% bovine serum albumin was incubated for 1 hour at room temperature. After washing with TBST, Abfrontier Westsave chemiluminescence detection kit was added dropwise to the membrane. The pattern was developed by x-ray film (Kodak). The film was scanned to convert to digital version for graphic analysis (Image J, NIH). Protein expression was quantified by evaluating grayscale values, quantified by mean optical density ± SD. Western blot results showed that the expression levels of CYP2E1 and CYP3A were significantly increased in the Con-A-induced liver injury model group compared with the normal group. The expression levels of CYP5A1 and CYP2E1 in the Composition A treatment group significantly decrease as compared with the model group (Figure 2). The results indicate that the mechanism of inducing liver injury by Con-A is related to CYP2E1 and CYP3A. The mechanism that the composition protects the liver from damage is related to CYP2E1. However, the composition has no effect on the expression of CYP5A1, but can increase the expression of CYP3A.

Accordingly, the results prove that Composition A can exhibit protection effects on the liver. Composition A can thus protect a subject from suffering from hepatitis such as autoimmune hepatitis. It is also recommended that the oral pharmaceutical composition of the present invention should not be heated or boiled before administration. Furthermore, it is suggested not to intake the oral pharmaceutical composition with hot food so as to maintain the therapeutic effect of the composition. Without intending to be limited by theory, it is believed that the components in the Composition A altered in the heat treatment process, e.g. some active components changed or evaporated, and thus Composition A100 produced relatively mild or less effects when compared with the untreated Composition A.

**Table 14. Effect of different doses of the oral pharmaceutical compositions on ALT levels**

| **Group** | **ALT (mU/mL)** | **vs. Control (%)** | **vs. Model (%)** |
|---|---|---|---|
| Control | 1.503±0.116 | ------ | 11.53 |
| Model | 13.03±0.204## | 866.9 | ------ |
| Ah | 10.83±0.249** | 720.6 | 83.12 |
| Am | 5.501±0.412** | 366.0 | 42.22 |
| Al | 4.856±0.363** | 323.1 | 37.27 |
| A100h | 12.80±0.262 | 851.6 | 98.23 |
| A100m | 12.21±0.634 | 812.4 | 93.71 |
| A100l | 10.30±0.578** | 685.3 | 79.05 |

| | | | |
|---|---|---|---|
| ## P<0.01 vs. control group; * P <0.05, ** P< 0.01 vs. model group. | | | |

**Table 15. Effect of different doses of the oral pharmaceutical compositions on IL-10 levels**

| **Group** | **IL-10 (pg/mL)** | **vs. Control (%)** | **vs. Model (%)** |
|---|---|---|---|
| Control | 100.38±2.93 | ------ | 583.27 |
| Model | 17.21±4.70## | 17.14 | ------ |
| Ah | 80.06±2.72** | 79.75 | 465.19 |
| Am | 55.93±9.50** | 55.72 | 324,99 |
| Al | 51.94±3.17** | 51.75 | 301.80 |
| A100h | 19.25±1.64 | 19.17 | 111.85 |
| A100m | 48.92±10.66** | 48.73 | 284.25 |
| A100l | 25.38±6.88* | 25.28 | 147.47 |

| | | | |
|---|---|---|---|
| ## P<0.01 vs. control group; * P <0.05, ** P< 0.01 vs. model group. | | | |

**Table 16. Effect of different doses of the oral pharmaceutical compositions on IFN-γ levels**

| **Group** | **IFN-γ (pg/mL)** | **vs. Control (%)** | **vs. Model (%)** |
|---|---|---|---|
| Control | 34.59±5.10 | ------ | 40.00 |
| Model | 86.47±5.46## | 249.99 | ------ |
| Ah | 39.64±6.24** | 114.60 | 45.84 |
| Am | 40.47+2.96** | 117.00 | 46.80 |
| Al | 82.80±3.54** | 239.38 | 95.76 |
| A100h | 80.79±13.41 | 233.56 | 93.43 |
| A100m | 77.49±3.39* | 224.02 | 89.61 |
| A100l | 80.04±6.44 | 231.40 | 92.56 |

| | | | |
|---|---|---|---|
| ## P<0.01 vs. control group; * P <0.05, ** P< 0.01 vs. model group. | | | |

**Table 17. Effect of different doses of the oral pharmaceutical compositions on TNF-α levels**

| **Group** | **TNF-α (pg/mL)** | **vs. Control (%)** | **vs. Model (%)** |
|---|---|---|---|
| Control | 320.17±17.67 | ------ | 28.43 |
| Model | 1126.17±121.69## | 351.74 | ------ |
| Ah | 750.99±72.96** | 234.56 | 66.69 |
| Am | 769.86±57.98** | 240.45 | 68.36 |
| Al | 749.27±12.00** | 234.02 | 66.53 |
| A100h | 949.53±191.44 | 296.59 | 84.32 |
| A100m | 985.94±84.43 | 307.94 | 87.54 |
| A100l | 1108.54±66.40 | 346.23 | 98.43 |

| | | | |
|---|---|---|---|
| ## P<0.01 vs. control group; * P <0.05, ** P< 0.01 vs. model group. | | | |

**Table 18. Effect of different doses of the oral pharmaceutical compositions on TG levels**

| **Group** | **TG (mmol/L)** | **vs. Control (%)** | **vs. Model (%)** |
|---|---|---|---|
| Control | 1.40±0.07 | ------- | 101.95 |
| Model | 1.37±0.08 | 98.09 | ------ |
| Ah | 1.40±0.03 | 100.00 | 101.95 |
| Am | 1.33±0.10 | 94.99 | 96.83 |
| Al | 1.34±0.04 | 95.70 | 97.57 |
| A100h | 1.35±0.06 | 96.90 | 98.78 |
| A100m | 1.39±0.03 | 99.76 | 101.70 |
| A100l | 1.45±0.07 | 103.58 | 105.60 |

| | | | |
|---|---|---|---|
| ## P<0.01 vs. control group; * P <0.05, ** P< 0.01 vs. model group. | | | |

**Table 19. Effect of different doses of the oral pharmaceutical compositions on TC levels**

| **Group** | **TC (mmol/L)** | **vs. Control (%)** | **vs. Model (%)** |
|---|---|---|---|
| Control | 2.98±0.03 | ------ | 100.00 |
| Model | 2.98±0.11 | 100.00 | ------ |
| Ah | 3.05±0.04 | 102.35 | 102.35 |
| Am | 3.00±0.11 | 100.67 | 100.67 |
| Al | 2,89±0.10 | 96.87 | 96.87 |
| A100h | 3.00±0.03 | 97.20 | 97.20 |
| A100m | 2.94±0.03 | 98.55 | 98.55 |
| A100l | 3.02±0.04 | 101.45 | 101.45 |

| | | | |
|---|---|---|---|
| ## P<0.01 vs. control group; * P <0.05, ** P<0.01 vs. model group. | | | |

**Table 20. Effect of different doses of the oral pharmaceutical compositions on PGE1 levels**

| **Group** | **PGE1 (pg/L))** | **vs. Control (%)** | **vs. Model (%)** |
|---|---|---|---|
| Control | 820.69±104.35 | ------ | 56.23 |
| Model | 1459.54±86.97## | 177.84 | ------ |
| Ah | 1004.04±88.59** | 122.34 | 68.79 |
| Am | 1003.29±66.77** | 122.25 | 68.74 |
| Al | 1249.37±44.75* | 152.23 | 85.60 |
| A100h | 1438.69±51.44 | 175.30 | 98.57 |
| A100m | 1452.69±121.76 | 177.01 | 99.53 |
| A100l | 1448.37±124.83 | 176.48 | 99.23 |

| | | | |
|---|---|---|---|
| ## P<0.01 vs. control group; * P <0.05, ** P< 0.01 vs. model group. | | | |

### EXAMPLE 5C

### Effects on animal model having chronic alcoholic liver induced by ethanol

KM mice were fed a nutritionally adequate liquid control diet for 5 days, treatment groups were fed with a control diet plus the composition as described above for 10 consecutive days. At day 11, mice in the model group and treatment groups were administered a single dose of 20% ethanol (5g/kg base on the total body weight of the mice), while mice in the control group were administered isocaloric dextrin maltose. The administrations were performed in the early morning. Blood samples were collected from the posterior vena cava. Plasma were obtained and stored at -20°C before subsequent biochemical analysis.

ELISA kits were used and standard curves were plotted using standard solutions. Table 21 to 27 show the cytokines levels obtained from the ELISA tests. The results show that the Composition A can significantly improve the ALT, IL-10, IFN-γ, TNF-α and PEG1 levels in the CCl4-induced acute liver injury. Composition A with a high dosage and a moderate dosage can exhibit effects on lowering TG and TC levels.

The results show that Composition A100 has certain effect on improving the IFN-γ levels while has no significant effect on other cytokines.

Accordingly, the results prove that Composition A can exhibit protection effects on the liver. Composition A can thus protect a subject from suffering from a chronic alcoholic liver injury. It is also recommended that the oral pharmaceutical composition of the present invention should not be heated or boiled before administration. Furthermore, it is suggested not to intake the oral pharmaceutical composition with hot food so as to maintain the therapeutic effect of the composition. Without intending to be limited by theory, it is believed that the components in the Composition A altered in the heat treatment process, e.g. some active components changed or evaporated, and thus Composition A100 produced relatively mild or less effects when compared with the untreated Composition A.

**Table 21. Effect of different doses of the oral pharmaceutical compositions on ALT levels**

| **Group** | **ALT (mU/mL)** | **vs. Control (%)** | **vs. Model (%)** |
|---|---|---|---|
| Control | 1.421±0.134 | ------ | 32.58 |
| Model | 4.361±0.198## | 306.9 | ------ |
| Ah | 1.958±0.223** | 137.8 | 44.90 |
| Am | 1.974±0.092** | 138.9 | 45.26 |
| Al | 1.883±0.479** | 132.5 | 43.18 |
| A100h | 4.023±0.229 | 283.1 | 92.25 |
| A100m | 3.126±0.175** | 220.0 | 71.68 |
| A100l | 3.075±0.162** | 216.4 | 70.51 |

| | | | |
|---|---|---|---|
| ## P<0.01 vs. control group; * P <0.05, ** P< 0.01 vs. model group. | | | |

**Table 22. Effect of different doses of the oral pharmaceutical compositions on IL-10 levels**

| **Group** | **IL-10 (pg/mL)** | **vs. Control (%)** | **vs. Model (%)** |
|---|---|---|---|
| Control | 98.29±8.57 | ------ | 521.98 |
| Model | 18.83±6.89## | 19.16 | ------ |
| Ah | 69.56±6.67** | 70.77 | 369.41 |
| Am | 56.04±2.91** | 57.01 | 297.61 |
| Al | 58.33±6.66** | 59.34 | 309.77 |
| A100h | 16.19±2.67 | 16.47 | 85.97 |
| A100m | 19.48±3.65 | 19.82 | 103.45 |
| A100l | 19.98±1.76 | 20.32 | 106.10 |

| | | | |
|---|---|---|---|
| ## P<0.01 vs. control group; * P <0.05, ** P< 0.01 vs. model group. | | | |

**Table 23. Effect of different doses of the oral pharmaceutical compositions on IFN-γ levels**

| **Group** | **IFN-γ (pg/mL)** | **vs. Control (%)** | **vs. Model (%)** |
|---|---|---|---|
| Control | 33.01±3.97 | ------ | 25.25 |
| Model | 130.71±10.00## | 395.97 | ------ |
| Ah | 60.83±8.10** | 184.28 | 46.54 |
| Am | 72.89±5.42** | 220.81 | 55.76 |
| Al | 74.22±3.22** | 224.84 | 56.78 |
| A100h | 118.18±5.97 | 358.01 | 90.41 |
| A100m | 98.61±6.25* | 298.73 | 75.44 |
| A100l | 109.38±12.09 | 331.35 | 83.68 |

| | | | |
|---|---|---|---|
| ## P<0.01 vs. control group; * P <0.05, ** P< 0.01 vs. model group. | | | |

**Table 24. Effect of different doses of the oral pharmaceutical compositions on TNF-α levels**

| **Group** | **TNF-α (pg/mL)** | **vs. Control (%)** | **vs. Model (%)** |
|---|---|---|---|
| Control | 316.20±20.20 | ------ | |
| Model | 447.73±25.50## | 141.59 | ------ |
| Ah | 360.49±19.12** | 114.01 | 80.51 |
| Am | 356.73±8.74** | 131.14 | 79.90 |
| Al | 416.47±4.44 | 131.71 | 93.02 |
| A100h | 407.79±14.04* | 128.97 | 91.08 |
| A100m | 458.83±19.34 | 145.11 | 102.48 |
| A100l | 433.71±23.45 | 137.17 | 96.87 |

| | | | |
|---|---|---|---|
| ## P<0.01 vs. control group; * P <0.05, ** P< 0.01 vs. model group. | | | |

**Table 25. Effect of different doses of the oral pharmaceutical compositions on TG levels**

| **Group** | **TG (mmol/L)** | **vs. Control (%)** | **vs. Model (%)** |
|---|---|---|---|
| Control | 1.39±0.02 | ------ | 74.06 |
| Model | 1.88±0.10## | 135.01 | ------ |
| Ah | 1.42±0.10** | 102.64 | 76.02 |
| Am | 1.59±0.06** | 114.87 | 85.08 |
| Al | 1.55±0.06 | 111.51 | 82.59 |
| A100h | 1.66±0.07 | 119.66 | 88.63 |
| A100m | 1.92±0.04 | 137.89 | 102.13 |
| A100l | 1.86±0.02 | 134.05 | 99.29 |

| | | | |
|---|---|---|---|
| ## P<0.01 vs. control group; * P <0.05, ** P< 0.01 vs. model group. | | | |

**Table 26. Effect of different doses of the oral pharmaceutical compositions on TC levels**

| **Group** | **TC (mmol/L)** | **vs. Control (%)** | **vs. Model (%)** |
|---|---|---|---|
| Control | 2.92±0.13 | ------ | 75.25 |
| Model | 3.88±0.10## | 132.88 | ------ |
| Ah | 2.97±0.06** | 101.60 | 76.46 |
| Am | 2.88±0.03** | 98.74 | 74.31 |
| Al | 3.66±0.10 | 125.34 | 94.33 |
| A100h | 3.76±0.18 | 128.65 | 96.82 |
| A100m | 3.88±0.03 | 133.00 | 100.09 |
| A100l | 3.91±0.08 | 133.79 | 100.69 |

| | | | |
|---|---|---|---|
| ## P<0.01 vs. control group; * P <0.05, ** P< 0.01 vs. model group. | | | |

**Table 27. Effect of different doses of the oral pharmaceutical compositions on PGE1 levels**

| **Group** | **PGE1 (pg/L))** | **vs. Control (%)** | **vs. Model (%)** |
|---|---|---|---|
| Control | 759.29±70.83 | ------ | 57.70 |
| Model | 1315.80±102.72## | 173.29 | ------ |
| Ah | 876.96±33.81** | 115.50 | 66.65 |
| Am | 804.47±33.80** | 105.95 | 61.14 |
| Al | 944.31±74.56** | 124.37 | 71.77 |
| A100h | 1388.65±120.17 | 182.89 | 105.54 |
| A100m | 1261.96±251.81 | 166.20 | 95.91 |
| A100l | 1083.78±62.48* | 142.74 | 82.37 |

| | | | |
|---|---|---|---|
| ## P<0.01 vs. control group; * P <0.05, ** P< 0.01 vs. model group. | | | |

### EXAMPLE 6

### Effects on patient suffering from hepatitis B

A dosage of 10 ml/day, i.e. around 2 teaspoons per day, of the oral pharmaceutical composition as prepared according to Example 1 was orally administered to a patient suffering from hepatitis B from May 24 2014. Before the oral administration, the AST level and ALT level of the patient were found to be 152 U/L and 349 U/L respectively. The level of gamma-glutamyl transferase was 31 U/L.

After taking the oral pharmaceutical composition daily for nearly 1 month, the AST level and ALT levels decreased to 30U/L and 51 U/L respectively at day 28. The level of gamma-glutamyl transferase was 18 U/L. At day 64, the AST level and ALT levels decreased to 23U/L and 30 U/L respectively. The level of gamma-glutamyl transferase was 15 U/L. Accordingly, the AST level and ALT level returned to normal level within 2 months after the oral administration of the composition.

The maximum values of AST and ALT levels at normal condition, i.e. health condition, are generally around 36 U/L and 45 U/L respectively. Accordingly, the oral pharmaceutical composition of the present invention is proved to be effective for treating a liver disease, in particular hepatitis.

### EXAMPLE 7

### Effect on patient suffering from arterial embolism

Based on a tomography report issued in February 2015, a patient suffering from arterial embolism was found to have the following conditions before any oral administration:
- small intermediate artery;
- moderate to severe (about 70%) non-calcified plaque in middle left anterior descending (LAD) artery; and
- moderate (25-50%) non-calcified plaque in distal LAD artery.

After taking the oral pharmaceutical composition as prepared according to Example 1 at a dosage of 10 ml/day, i.e. around 2 teaspoons per day, for nearly 5 months, the patient took another computed tomography scan in June 2015. The results show that the size of the intermediate artery returned to normal condition and there were only soft plaque in middle LAD artery. The total calcium score was 0.

Accordingly, it proves that oral pharmaceutical composition of the present invention is capable of treating a patient suffering from cardiovascular disease, e.g. an arterial embolism. It will be appreciated by the person skilled in the art to apply the oral pharmaceutical composition of the present invention in treating and/or preventing various diseases, such as those associated with liver disorders and cardiovascular disorders.

It will be appreciated a number of alternative embodiments have been described herein. It will be appreciated by persons skilled in the art that the alternative embodiments described can be used in part or wholly with any of the embodiments described. It will also be appreciated that portions or elements that are known in the prior art or known to persons skilled in the art have not been explicitly described. It will also be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. An oral pharmaceutical composition comprising an essential oil portion and a carrier oil portion, wherein the essential oil portion consists essentially of
• from about 20 vol.-% to about 50 vol.-% of the essential oil derived from *Citrus bergamia* based on the total volume of the essential oil portion;
• from about 20 vol.-% to about 50 vol.-% of the essential oil derived from *Matricaria chamomilla* based on the total volume of the essential oil portion;
• from about 10 vol.-% to about 30 vol.-% of the essential oil derived from *Origanum vulgare* based on the total volume of the essential oil portion;
• from about 5 vol.-% to about 20 vol.-% of the essential oil derived from *Juniperus communis* based on the total volume of the essential oil portion; and
• from about 5 vol.-% to about 20 vol.-% of the essential oil derived from *Rosmarinus officinalis* based on the total volume of the essential oil portion; and
wherein a carrier oil portion comprises a fatty acid.

2. The oral pharmaceutical composition according to claim 1, wherein the amount of the essential oil portion is from about 0.1 vol.-% to about 20 vol.-% based on the total volume of the oral pharmaceutical composition.

3. The oral pharmaceutical composition according to claim 1, wherein the amount of the carrier oil portion is from about 80 vol.-% to about 99.9 vol.-% based on the total volume of the oral pharmaceutical composition; or wherein the carrier oil portion comprises at least two fatty acids selected from the group consisting of oleic acid, vaccenic acid, linoleic acid, α-linolenic acid, and γ-linolenic acid.

4. The oral pharmaceutical composition according to claim 1, wherein the oral pharmaceutical composition comprises at least 5% of limonene, at least 5% of 1,8-cineole and at least 5% of carvacrol based on the volume of the oral pharmaceutical composition; or wherein the oral pharmaceutical composition comprises vaccenic acid in an amount of from about 0.5 wt.-% to 10 wt.-% based on the total amount of fatty acids in the oral pharmaceutical composition.

5. The oral pharmaceutical composition according to claim 1, wherein the carrier oil portion comprises a vegetable fatty acid derived from a plant, and wherein the plant's genus is selected from the group consisting of *Cannabis, Sesamum, Prunus dulcis,* and *Rosmarinus.*

6. The oral pharmaceutical composition according to claim 1, wherein the oral pharmaceutical composition further comprises a polymer, and wherein the essential oil portion and the carrier oil portion are encapsulated in the polymer, and optionally the polymer is a polysaccharide.

7. The oral pharmaceutical composition according to claim 6, wherein the essential oil portion and the carrier oil portion are coated with a coating film and wherein the coating film consists essentially of the polymer.

8. The oral pharmaceutical composition according to claim 1 for use in the treatment or prevention of a disease.

9. The composition for use according to claim 8, wherein the disease is a liver disease or a cardiovascular disease, and wherein the liver disease is selected from the group consisting of hepatitis, alcoholic liver disease, fatty liver disease, hereditary disease, fascilasis, cirrhosis, liver cancer and a combination thereof, and the cardiovascular disease is selected from the group consisting of atherosclerosis, restenosis, coronary arterial disease, peripheral arterial disease, aneurysm, thrombosis, embolism, myocardial infarction, ischemia and a combination thereof.

10. A food supplement composition comprising an essential oil portion and a carrier oil portion, wherein the essential oil portion consists essentially of
• from about 20 vol.-% to about 50 vol.-% of the essential oil derived from *Citrus bergamia* based on the total volume of the essential oil portion;
• from about 20 vol.-% to about 50 vol.-% of the essential oil derived from *Matricaria chamomilla* based on the total volume of the essential oil portion;
• from about 10 vol.-% to about 30 vol.-% of the essential oil derived from *Origanum vulgare* based on the total volume of the essential oil portion;
• from about 5 vol.-% to about 20 vol.-% of the essential oil derived from *Juniperus communis* based on the total volume of the essential oil portion; and
• from about 5 vol.-% to about 20 vol.-% of the essential oil derived from *Rosmarinus officinalis* based on the total volume of the essential oil portion; and
wherein a carrier oil portion comprises a fatty acid.

11. The food supplement composition according to claim 10, wherein the amount of the essential oil portion is from about 0.1 vol.-% to about 5 vol.-% based on the total volume of the food supplement composition.

12. The food supplement composition according to claim 10, wherein the amount of the carrier oil portion is from about 95 vol.-% to about 99.9 vol.-% based on the total volume of the food supplement composition; or wherein the carrier oil portion comprises at least two fatty acids selected from the group consisting of oleic acid, vaccenic acid, linoleic acid, α-linolenic acid, and γ-linolenic acid.

13. The food supplement composition according to claim 10, wherein the food supplement composition comprises at least 5% of limonene, at least 5% of 1,8-cineole and at least 5% of carvacrol based on the volume of the food supplement composition.

14. The food supplement composition according to claim 10, wherein the carrier oil portion comprises a vegetable fatty acid derived from a plant, and wherein the plant's genus is selected from the group consisting of *Cannabis, Sesamum, Prunus dulcis,* and *Rosmarinus.*

15. The food supplement composition according to claim 10, wherein the food supplement composition comprises
• from about 0.5 wt.-% to 10 wt.-% of vaccenic acid based on the total amount of fatty acids in the food supplement composition;
• from about 20 wt.-% to 60 wt.-% of oleic acid based on the total amount of fatty acids in the food supplement composition;
• from about 20 wt.-% to 60 wt.-% of linoleic acid based on the total amount of fatty acids in the food supplement composition;
• from about 0.5 wt.-% to 10 wt.-% of α-linolenic acid based on the total amount of fatty acids in the food supplement composition; and
• from about 0.5 wt.-% to 10 wt.-% of γ-linolenic acid based on the total amount of fatty acids in the food supplement composition.

## Patentansprüche

1. Orale pharmazeutische Zusammensetzung, umfassend einen Anteil essentiellen Öls und einen Trägerölanteil, wobei der Anteil essentiellen Öls im Wesentlichen besteht aus:
• bezogen auf das Gesamtvolumen des Anteils essentiellen Öls von etwa 20 Vol.-% bis etwa 50 Vol.-% von *Citrus bergamia* abgeleiteten essentiellen Öls;
• bezogen auf das Gesamtvolumen des Anteils essentiellen Öls von etwa 20 Vol.-% bis etwa 50 Vol.-% von *Matricaria chamomilla* abgeleiteten essentiellen Öls;
• bezogen auf das Gesamtvolumen des Anteils essentiellen Öls von etwa 10 Vol.-% bis etwa 30 Vol.-% von *Origanum vulgare* abgeleiteten essentiellen Öls;
• bezogen auf das Gesamtvolumen des Anteils essentiellen Öls von etwa 5 Vol.-% bis etwa 20 Vol.-% von *Juniperus communis* abgeleiteten essentiellen Öls; und
• bezogen auf das Gesamtvolumen des Anteils essentiellen Öls von etwa 5 Vol.-% bis etwa 20 Vol.-% von *Rosmarinus officinalis* abgeleiteten essentiellen Öls; und wobei ein Trägerölanteil eine Fettsäure umfasst.

2. Orale pharmazeutische Zusammensetzung nach Anspruch 1, wobei die Menge des Anteils essentiellen Öls bezogen auf das Gesamtvolumen der oralen pharmazeutischen Zusammensetzung von etwa 0,1 Vol.-% bis etwa 20 Vol.-% beträgt.

3. Orale pharmazeutische Zusammensetzung nach Anspruch 1, wobei die Menge des Trägerölanteils bezogen auf das Gesamtvolumen der oralen pharmazeutischen Zusammensetzung von etwa 80 Vol.-% bis etwa 99,9 Vol.-% beträgt; oder wobei der Trägerölanteil mindestens zwei Fettsäuren umfasst, die ausgewählt sind aus der Gruppe bestehend aus Oleinsäure, Vaccensäure, Linolsäure, α-Linolsäure und *γ*-Linolsäure.

4. Orale pharmazeutische Zusammensetzung nach Anspruch 1, wobei die orale pharmazeutische Zusammensetzung bezogen auf das Volumen der oralen pharmazeutischen Zusammensetzung mindestens 5% Limonen, mindestens 5% von 1,8-Cineol und mindestens 5% Carvacrol umfasst; oder wobei die orale pharmazeutische Zusammensetzung bezogen auf die Gesamtmenge von Fettsäuren in der oralen pharmazeutischen Zusammensetzung Vaccensäure in einer Menge von etwa 0,5 Gew.-% bis 10 Gew.-% umfasst.

5. Orale pharmazeutische Zusammensetzung nach Anspruch 1, wobei der Trägerölanteil eine pflanzliche Fettsäure umfasst, die abgeleitet ist von einer Pflanze, und wobei die Gattung der Pflanze ausgewählt ist aus der Gruppe bestehend aus *Cannabis, Sesamum, Prunus dulcis* und *Rosmarinus.*

6. Orale pharmazeutische Zusammensetzung nach Anspruch 1, wobei die orale pharmazeutische Zusammensetzung ferner ein Polymer umfasst; und wobei der Anteil essentiellen Öls und der Trägerölanteil in dem Polymer gekapselt sind und wahlweise wobei das Polymer ein Polysaccharid ist.

7. Orale pharmazeutische Zusammensetzung nach Anspruch 6, wobei der Anteil essentiellen Öls und der Trägerölanteil mit einem Überzugsfilm überzogen sind und wobei der Überzugsfilm im Wesentlichen aus dem Polymer besteht.

8. Orale pharmazeutische Zusammensetzung nach Anspruch 1 zur Verwendung in der Behandlung oder Verhütung einer Erkrankung.

9. Zusammensetzung zu Verwendung nach Anspruch 8, wobei die Erkrankung eine Lebererkrankung oder eine kardiovaskuläre Erkrankung ist und wobei die Lebererkrankung ausgewählt ist aus der Gruppe bestehend aus Hepatitis, alkoholischer Lebererkrankung, Fettlebererkrankung, Erbkrankheit, Fascilasis, Zirrhose, Leberkrebs und einer Kombination davon, und die kardiovaskuläre Erkrankung ausgewählt ist aus der Gruppe bestehend aus Atherosklerose, Restenose, koronarer Herzkrankheit, peripherer arterieller Erkrankung, Aneurysma, Thrombose, Embolie, Myokardinfarkt, Ischämie und Kombinationen davon.

10. Nahrungsergänzungsmittel-Zusammensetzung, umfassend einen Anteil essentiellen Öls und einen Trägerölanteil, wobei der Anteil essentiellen Öls im Wesentlichen besteht aus:
• bezogen auf das Gesamtvolumen des Anteils essentiellen Öls von etwa 20 Vol.-% bis etwa 50 Vol.-% von *Citrus bergamia* abgeleiteten essentiellen Öls;
• bezogen auf das Gesamtvolumen des Anteils essentiellen Öls von etwa 20 Vol.-% bis etwa 50 Vol.-% von *Matricaria chamomilla* abgeleiteten essentiellen Öls;
• bezogen auf das Gesamtvolumen des Anteils essentiellen Öls von etwa 10 Vol.-% bis etwa 30 Vol.-% von *Origanum vulgare* abgeleiteten essentiellen Öls;
• bezogen auf das Gesamtvolumen des Anteils essentiellen Öls von etwa 5 Vol.-% bis etwa 20 Vol.-% von *Juniperus communis* abgeleiteten essentiellen Öls; und
• bezogen auf das Gesamtvolumen des Anteils essentiellen Öls von etwa 5 Vol.-% bis etwa 20 Vol.-% von *Rosmarinus officinalis* abgeleiteten essentiellen Öls; und wobei ein Trägerölanteil eine Fettsäure umfasst.

11. Nahrungsergänzungsmittel-Zusammensetzung nach Anspruch 10, wobei die Menge des Trägerölanteils bezogen auf das Gesamtvolumen der Nahrungsergänzungsmittel-Zusammensetzung von etwa 0,1 Vol.-% bis etwa 5 Vol.-% beträgt.

12. Nahrungsergänzungsmittel-Zusammensetzung nach Anspruch 10, wobei die Menge des Trägerölanteils bezogen auf das Gesamtvolumen der Nahrungsergänzungsmittel-Zusammensetzung von etwa 95 Vol.-% bis etwa 99,9 Vol.-% beträgt; oder wobei der Trägerölanteil mindestens zwei Fettsäuren umfasst, die ausgewählt sind aus der Gruppe bestehend aus Oleinsäure, Vaccensäure, Linolsäure, α-Linolsäure und *γ*-Linolsäure.

13. Nahrungsergänzungsmittel-Zusammensetzung nach Anspruch 10, wobei die Nahrungsergänzungsmittel-Zusammensetzung bezogen auf das Volumen der Nahrungsergänzungsmittel-Zusammensetzung mindestens von 5% Limonen, mindestens 5% 1,8-Cineol und mindestens 5% Carvacrol umfasst.

14. Nahrungsergänzungsmittel-Zusammensetzung nach Anspruch 10, wobei der Trägerölanteil eine pflanzliche Fettsäure umfasst, die abgeleitet ist von einer Pflanze, und wobei die Gattung der Pflanze ausgewählt ist aus der Gruppe bestehend aus *Cannabis, Sesamum, Prunus dulcis* und *Rosmarinus.*

15. Nahrungsergänzungsmittel-Zusammensetzung nach Anspruch 10, wobei die Nahrungsergänzungsmittel-Zusammensetzung umfasst:
• bezogen auf die Gesamtmenge von Fettsäuren in der Nahrungsergänzungsmittel-Zusammensetzung von etwa 0,5 Gew.-% bis 10 Gew.-% Vaccensäure;
• bezogen auf die Gesamtmenge von Fettsäuren in der Nahrungsergänzungsmittel-Zusammensetzung von etwa 20 Gew.-% bis etwa 60 Gew.-% Oleinsäure;
• bezogen auf die Gesamtmenge von Fettsäuren in der Nahrungsergänzungsmittel-Zusammensetzung von etwa 20 Gew.-% bis etwa 60 Gew.-% Linolsäure;
• bezogen auf die Gesamtmenge von Fettsäuren in der Nahrungsergänzungsmittel-Zusammensetzung von etwa 0,5 Gew.-% bis 10 Gew.-% α-Linolsäure; und
• bezogen auf die Gesamtmenge von Fettsäuren in der Nahrungsergänzungsmittel-Zusammensetzung von etwa 0,5 Gew.-% bis 10 Gew.-% γ-Linolsäure.

## Revendications

1. Composition pharmaceutique orale comprenant une partie d'huile essentielle et une partie d'huile de support, dans laquelle la partie d'huile essentielle est constituée essentiellement :
• d'environ 20 % en vol. à environ 50 % en vol. d'huile essentielle dérivée de *Citrus bergamia,* rapporté au volume total de la partie d'huile essentielle,
• d'environ 20 % en vol. à environ 50 % en vol. d'huile essentielle dérivée de *Matricaria chamomilla,* rapporté au volume total de la partie d'huile essentielle,
• d'environ 10 % en vol. à environ 30 % en vol. d'huile essentielle dérivée de *Origanum vulgare,* rapporté au volume total de la partie d'huile essentielle,
• d'environ 5 % en vol. à environ 20 % en vol. d'huile essentielle dérivée de *Juniperus communis,* rapporté au volume total de la partie d'huile essentielle, et
• d'environ 5 % en vol. à environ 20 % en vol. d'huile essentielle dérivée de *Rosmarinus officinalis,* rapporté au volume total de la partie d'huile essentielle, et
dans laquelle une partie d'huile de support comprend un acide gras.

2. Composition pharmaceutique orale selon la revendication 1, dans laquelle la quantité de la partie d'huile essentielle est d'environ 0,1 % en volume à environ 20 % en volume rapporté au volume total de la composition pharmaceutique orale.

3. Composition pharmaceutique orale selon la revendication 1, dans laquelle la quantité de la partie d'huile de support est d'environ 80 % en volume à environ 99,9 % en volume rapporté au volume total de la composition pharmaceutique orale, ou dans laquelle la partie d'huile de support comprend au moins deux acides gras sélectionnés dans le groupe constitué de l'acide oléique, l'acide vaccénique, l'acide linoléique, l'acide α-linolénique, et l'acide γ-linolénique.

4. Composition pharmaceutique orale selon la revendication 1, où la composition pharmaceutique orale comprend au moins 5 % de limonène, au moins 5 % de 1,8-cinéole et au moins 5 % de carvacrol rapporté au volume de la composition pharmaceutique orale, ou où la composition pharmaceutique orale comprend de l'acide vaccénique en une quantité d'environ 0,5 % en poids à 10 % en poids rapporté à la quantité totale d'acides gras dans la composition pharmaceutique orale.

5. Composition pharmaceutique orale selon la revendication 1, dans laquelle la partie d'huile de support comprend un acide gras végétal dérivé d'une plante, et dans laquelle le genre de la plante est sélectionné dans le groupe constitué de *Cannabis, Sesamum, Prunus dulcis* et *Rosmarinus.*

6. Composition pharmaceutique orale selon la revendication 1, où la composition pharmaceutique orale comprend en outre un polymère, et dans laquelle la partie d'huile essentielle et la partie d'huile de support sont encapsulées dans le polymère, et optionnellement dans laquelle le polymère est un polysaccharide.

7. Composition pharmaceutique orale selon la revendication 6, dans laquelle la partie d'huile essentielle et la partie d'huile de support sont enrobées avec un film d'enrobage et dans laquelle le film d'enrobage est constitué essentiellement du polymère.

8. Composition pharmaceutique orale selon la revendication 1 pour une utilisation dans le traitement ou la prévention d'une maladie.

9. Composition pour une utilisation selon la revendication 8, dans laquelle la maladie est une maladie hépatique ou une maladie cardiovasculaire, et dans laquelle la maladie hépatique est sélectionnée dans le groupe constitué de : une hépatite, une maladie alcoolique du foie, une stéatose hépatique, une maladie héréditaire, une fasciolase, une cirrhose, un cancer du foie et une combinaison de ceux-ci et la maladie cardiovasculaire est sélectionnée dans le groupe constitué de : une athérosclérose, une resténose, une maladie coronarienne, une maladie artérielle périphérique, un anévrisme, une thrombose, une embolie, un infarctus du myocarde, une ischémie et une combinaison de ceux-ci.

10. Composition de complément alimentaire comprenant une partie d'huile essentielle et une partie d'huile de support, dans laquelle la partie d'huile essentielle est constituée essentiellement :
• d'environ 20 % en vol. à environ 50 % en vol. d'huile essentielle dérivée de *Citrus bergamia,* rapporté au volume total de la partie d'huile essentielle,
• d'environ 20 % en vol. à environ 50 % en vol. d'huile essentielle dérivée de *Matricaria chamomilla,* rapporté au volume total de la partie d'huile essentielle,
• d'environ 10 % en vol. à environ 30 % en vol. d'huile essentielle dérivée *d'Origanum vulgare,* rapporté au volume total de la partie d'huile essentielle,
• d'environ 5 % en vol. à environ 20 % en vol. d'huile essentielle dérivée de *Juniperus communis,* rapporté au volume total de la partie d'huile essentielle, et
• d'environ 5 % en vol. à environ 20 % en vol. d'huile essentielle dérivée de *Rosmarinus officinalis,* rapporté au volume total de la partie d'huile essentielle, et
dans laquelle une partie d'huile de support comprend un acide gras.

11. Composition de complément alimentaire selon la revendication 10, dans laquelle la quantité de la partie d'huile essentielle est d'environ 0,1 % en volume à environ 5 % en volume rapporté au volume total de la composition de complément alimentaire.

12. Composition de complément alimentaire selon la revendication 10, dans laquelle la quantité de la partie d'huile de support est d'environ 95 % en volume à environ 99,9 % en volume rapporté au volume total de la composition de complément alimentaire, ou dans laquelle la partie d'huile de support comprend au moins deux acides gras sélectionnés dans le groupe constitué de l'acide oléique, l'acide vaccénique, l'acide linoléique, l'acide α-linolénique, et l'acide γ-linolénique.

13. Composition de complément alimentaire selon la revendication 10, où la composition de complément alimentaire comprend au moins 5 % de limonène, au moins 5 % de 1,8-cinéole et au moins 5 % de carvacrol rapporté au volume de la composition de complément alimentaire.

14. Composition de complément alimentaire selon la revendication 10, dans laquelle la partie d'huile de support comprend un acide gras végétal dérivé d'une plante, et dans laquelle le genre de la plante est sélectionné dans le groupe constitué de *Cannabis, Sesamum, Prunus dulcis* et *Rosmarinus.*

15. Composition de complément alimentaire selon la revendication 10, dans laquelle la composition de complément alimentaire comprend
• d'environ 0,5 % en poids à 10 % en poids d'acide vaccénique rapporté à la quantité totale des acides gras dans la composition de complément alimentaire,
• d'environ 20 % en poids à 60 % en poids d'acide oléique rapporté à la quantité totale des acides gras dans la composition de complément alimentaire,
• d'environ 20 % en poids à 60 % en poids d'acide linoléique rapporté à la quantité totale des acides gras dans la composition de complément alimentaire,
• d'environ 0,5 % en poids à 10 % en poids d'acide α-linolénique rapporté à la quantité totale des acides gras dans la composition de complément alimentaire, et
• d'environ 0,5 % en poids à 10 % en poids d'acide γ-linolénique rapporté à la quantité totale des acides gras dans la composition de complément alimentaire,
